(19) Europäisches Patentamt
European Patent Office
Office européen des brevets

(11) Publication number: 0 050 864
B1

(12) EUROPEAN PATENT SPECIFICATION

(45) Date of publication of patent specification: 09.01.85

(51) Int. Cl.[4]: B 01 D 13/04, C 08 J 5/22

(21) Application number: 81108827.7

(22) Date of filing: 23.10.81

# (54) A hydrophilic cationic charge modified microporous membrane, a process for producing it and its use.

(30) Priority: 27.10.80 US 201366

(43) Date of publication of application:
05.05.82 Bulletin 82/18

(45) Publication of the grant of the patent:
09.01.85 Bulletin 85/02

(84) Designated Contracting States:
CH DE FR GB IT LI

(56) References cited:
DE-A-2 622 805
FR-A-2 391 752
FR-A-2 452 310
GB-A-1 411 843
US-A-3 567 630
US-A-4 005 012
US-A-4 208 455

(73) Proprietor: AMF INCORPORATED
World Headquarters 777 Westchester Avenue
White Plains New York 10604 (US)

(72) Inventor: Ostreicher, Eugene A.
Reservation Road
Farmington, Conn. 06032 (US)
Inventor: Knight, Rodney, A.
147 Lone Oak Drive
New Milford, Conn. 06776 (US)
Inventor: Fiore, Joseph V.
205 Steiner St.
Fairfield, Conn. 06430 (US)
Inventor: Emond, George T.
156 Meander Lane
Southington, Conn. 06489 (US)
Inventor: Hou, Kenneth C.
6314 Mallard Point
San Antonio Texas 78239 (US)

(74) Representative: Patentanwälte Grünecker, Dr. Kinkeldey, Dr. Stockmair, Dr. Schumann, Jakob, Dr. Bezold, Meister, Hilgers, Dr. Meyer-Plath
Maximilianstrasse 58
D-8000 München 22 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 050 864 B1

Courier Press, Leamington Spa, England.

## Description

This invention relates to a hydrophilic cationic charge modified microporous membrane comprising a hydrophilic organic polymeric microporous membrane having a micro-structure throughout said membrane and a primary charge modifying agent bonded to substantially all of the membrane micro-structure without substantial pore size reduction or pore blockage. This invention further relates to a process for producing said membrane; and it relates to the use of said membrane.

Microporous membranes are well known in the art. For example, U.S. Patent No. 3,876,738 describes a process for preparing a microporous membrane, for example, by quenching a solution of a film forming polymer in a non-solvent system for the polymer. European Patent Application 0 005 536 describes a similar process.

Other processes for producing microporous membranes are described, for example, in U.S. Patents: 3,642,668 and 4,247,498.

Microporous membranes, for example, made of nylon, are commercially available. Another commercially significant membrane is made of polyvinylidene fluoride. This membrane is probably produced according to U.S. Patents 4,203,847 and 4,203,848. Such membranes are advertised as useful for the sterile filtration of pharmaceuticals, e.g. removal of microorganisms.

From FR—A—2452310 there is known a microporouscellulosic membrane that has been cationically modified with a polyamido -polyamine epichlorhydrine resin. This reference does not describe treating a polymeric microporous membrane, but cellulosic fibers and particulate filter elements. The hydrophilic cationic charge modified microporous membrane of the invention belongs to a technical field which is clearly different from the filter material described in FR—A—2452310.

It is therefore object of the present invention to provide a microporous membrane which is particularly suitable for the filtration of biological or parenteral liquids.

Therefore a hydrophilic cationic charge modified microporous membrane, comprising a hydrophilic organic polymeric microporous membrane having a micro-structure throughout said membrane and a primary charge modifying agent bonded to substantially all of the membrane micro-structure without substantial pore size reduction or pore blockage is provided, which is characterised in the primary charge modifying agent being a water soluble organic polymer having a molecular weight greater than about 1000, wherein each monomer thereof has at least one epoxide group capable of bonding to the surface of the membrane and at least one tertiary amine or quaternary ammonium group.

The new membrane is isotropic and has low extractables. It is sanitisable or sterilizable. The microporous membrane according to the invention has an enhanced adsorptive sequestration capacity and an enhanced capture potential and is capable of capturing anionic particulate contaminant of a size smaller than the effective pore size of the membrane.

Preferably, a portion of the epoxy groups on the primary charge modifying agent are bonded to a secondary charge modifying agent selected from the group consisting of:

i) aliphatic amines which are polyamines having at least one primary amine or at least two secondary amines; and
ii) aliphatic amines having at least one secondary amine and a carboxyl or hydroxyl substituent.

The invention is further directed to a process for producing a hydrophilic cationic modified microporous membrane, which comprises applying to a hydrophilic organic polymeric microporous membrane having a microstructure throughout said membrane, a charge modifying amount of a primary cationic charge modifying agent bonded to substantially all of the membrane micro-structure, without substantial pore size reduction or pore blockage. Preferably, the process for charge modifying the microporous membrane comprises contacting the membrane with aqueous solutions of the charge modifying agents.

The preferred microporous membrane is nylon, the preferred primary and secondary charge modifying agents are respectively, polyamido-polyamine epichlorohydrin and tetraethylene pentamine.

The invention is further directed to the above-mentioned process which is characterised in further chemically treating the nylon membrane to provide enhanced ninhydrin response evidencing free amino functionality whereby the responsiveness to charge modification is improved.

The charge modified microporous membrane of this invention may be used for the filtration of fluids, particularly parenteral or biological liquids, except the use of the new membrane for medical treatment.

In the drawings

Fig. 1 is a graphical representation showing performance data, i.e. filtration efficiency, for a 0.2 micrometer membrane treated in accordance with the invention as compared to an untreated membrane, both challenged with 0.109 contaminant at .002 lpm/cm$^2$ (See Example VII); and

Figs. 2 and 3 are Scanning Electron Microscope (SEM) photos of the cationic charge modified membrane of this invention challenged with contaminant (See Example VII).

The cationic charge modified microporous membrane of this invention is produced from a hydrophilic organic polymeric microporous membrane. Such membranes are well known in the art.

By the use of the term "microporous membrane" as used herein, it is meant a substantially symmetrical, isotropic porous membrane having a pore size of at least .05 microns or larger or an initial bubble point (IBP), as that term is used herein, in water of less than 8,3 bar. A maximum pore size useful for this invention is about 1.2 micrometres or an IBP of greater than about 0,7 bar. By "symmetrical" it is meant that the pore structure is substantially the same on both sides of the membrane. A number of commercially available membranes not encompassed by the term "microporous membrane" are "asymmetric", i.e. having one side formed with a very light thin layer which is supported by a much more porous open structure. By the use of the term "isotropic", it is meant the membrane has a uniform pore structure throughout the membrane.

By the use of the term "hydrophilic" in describing the microporous membrane, it is meant a membrane which adsorbs or absorbs water. Generally, such hydrophilicity is produced by a sufficient amount of hydroxyl (OH—), carboxyl (—COOH) amino (—$NH_2$) and/or similar functional groups on the surface of the membrane. Such groups assist in the adsorption and/or absorption of the water onto the membrane. Such hydrophilicity of the membrane is a necessary element of this invention to provide the adequate bonding of the primary charge modifying agent through the epoxide substituent to the microporous membrane.

A preferred microporous membrane is one produced from nylon. The term "nylon" is intended to embrace film forming polyamide resins including copolymers and terpolymers which include the recurring amido grouping.

While, generally, the various nylon or polyamide resins are all copolymers of a diamine and a dicarboxylic acid, or homopolymers of a lactam of an amino acid, they vary widely in crystallinity or solid structure, melting point, and other physical properties. Preferred nylons for use in this invention are copolymers of hexamethylene diamine and adipic acid (nylon 66), copolymers of hexamethylene diamine and sebacic acid (nylon 610), and homopolymers of poly-o-caprolactam (nylon 6).

Alternatively, these preferred polyamide resins have a ratio of methylene ($CH_2$) to amide (NHCO) groups within the range 5:1 to 8:1, most preferably 5:1 to 7:1. Nylon 6 and nylon 66 each have a ratio of 6:1, whereas nylon 610 has a ratio of 8:1.

The nylon polymers are available in a wide variety of grades, which vary appreciably with respect to molecular weight, within the range from 15,000 to 42,000 and in other characteristics.

The highly preferred species of the units composing the polymer chain is polyhexamethylene adipamide, i.e. nylon 66, and molecular weights in the range above about 30,000 are preferred. Polymers free of additives are generally preferred, but the addition of antioxidants or similar additives may have benefit under some conditions.

The preferred membrane substrates are produced by the method disclosed in U.S. Patent No. 3,876,738 to *Marinaccio et al.* Another method of producing such membranes is described in European Patent Application No. 0 005 536 to *Pall.*

Additionally, any of the hydrophilic microporous membranes commercially available, for example, membranes made of nylon or polyvinylidene fluoride, and cellulose acetate/nitrate membranes produced by numerous companies, having characteristics potentially suitable for fine filtration of fluids, particularly aqueous systems, are suitable for treatment in accordance with this invention.

The primary charge modifying agent used in this invention is a water soluble organic polymer having a molecular weight greater than about 1000, wherein the monomer has at least one epoxide substituent capable of bonding to the surface of the membrane and at least one tertiary amine or quarternary ammonium group capable of providing a cationic charge site.

The primary charge modifying agent is bonded to substantially all of the membrane microstructure, without substantial pore size reduction or pore blockage. By the use of the term "bonded" it is meant that the charge modifying agent(s) are sufficiently attached to the membrane and/or to each other so that they will not significantly extract under the intended conditions of use. By the use of the term "substantially all of the wetted surface" as used herein it is meant all of the external surface and internal pore surfaces which are wetted by a fluid passing through the membrane or in which the membrane is immersed.

The preferred charge modifier is selected from the class of polyamido-polyamine epichlorohydrin cationic resins, in particular, those described in U.S. Patents 2,926,116, 2,926,154, 3,224,986, 3,311,594, 3,332,901, 3,382,096 and 3,761,350.

Broadly, these preferred charge modifiers (hereinafter "polyamido-polyamine epichlorohydrin") are produced by reacting a long chain polyamide with epichlorohydrin, i.e. 1-chloro-2,3-epoxypropane having the formula:

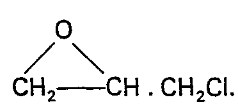

The polyamide may be derived from the reaction of a polyalkylene polyamine and a saturated

aliphatic dibasic carboxylic acid containing from 3 to 10 carbon atoms. The polyamide produced is water soluble and contains the recurring groups:

$$—NH(C_nH_{2n}HN)_x—CORCO—$$

where n and x are each 2 or more and R is the divalent hydrocarbon radical of the dicarboxylic acid. This polyamide is then reacted with epichlorohydrin to form the preferred water soluble charge modifiers used in this invention.

The dicarboxylic acids which may be used in preparing the polyamides are the saturated aliphatic dicarboxylic acids containing from 3 to 10 carbon atoms each as malonic, succinic, glutaric, adipic, azelaic and the like. Blends, of two or more of the saturated carboxylic acids may also be used.

A variety of polyalkylene polyamines including polyethylene polyamines, polypropylene polyamines, polybutylene polyamides and so on may be employed. More specifically, the polyalkylene polyamines are polyamines, containing two primary amine groups and at least one secondary amine group in which the nitrogen atoms are linked together by groups of the formula $—C_nH_{2n}—$, where n is a small integer greater than unity and the number of such groups in the molecule ranges from two up to about eight. The nitrogen atoms may be attached to adjacent carbon atoms in the group $—C_nH_{2n}—$ or to carbon atoms further apart, but not to the same carbon atom. Polyamines such as diethylenetriamine, triethylenetetramine, tetraethylene-pentamine, dipropylenetriamine, and the like, and mixtures thereof may be used. Generally, these polyalkylene polyamines have the general formula:

$$H_2[(C_nH_{2n})NH]_yC_nH_{2n}NH_2$$

wherein n is an integer of at least 2 and y is an integer of 1 to 7.

In carrying out the reaction of the polyalkylene polyamine with the acid, it is preferred to use an amount of dicarboxylic acid sufficient to react substantially completely with the primary amine groups of the polyalkylene polyamine but insufficient to react with the secondary amine groups to any substantial extent. The polyamide produced is then reacted with the epichlorohydrin to form the preferred polyamido-polyamine epichlorohydrin charge modifying agent. Typically, in the polyamide-epichlorohydrin reaction it is preferred to use sufficient epichlorohydrin to convert all of the secondary amine groups to tertiary amine groups, and/or quaternary ammonium groups (including cyclic structures). Generally, however from 0.5 mol to 1.8 moles of epichlorohydrin for each secondary amine group of the polyamide may be used.

The epichlorohydrin may also be reacted with a polyaminoureylene containing tertiary amine nitrogens to produce the primary charge modifying agents which may be utilized in this invention.

Other suitable charge modifying agents of the foregoing type may be produced by reacting a heterocyclic dicarboxylic acid with a diamine or polyalkylene polyamine and reacting the resultant product with epichlorohydrin.

The polyamido-polyamine epichlorohydrin cationic resins are available commercially.

Most preferably the polyamido-polyamine epichlorohydrin is a resin, wherein the charged nitrogen atom forms part of a heterocyclic grouping, and is bonded through methylene to a depending, reactive epoxide group.

Each monomer group in the most preferred resin has the general formula:

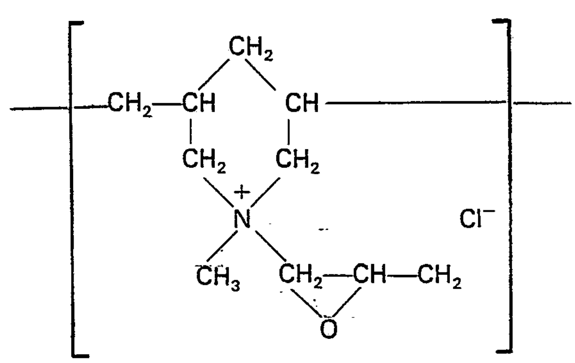

Other preferred polyamido-polyamino epichlorohydrin cationic resins have monomer groups of the general formula:

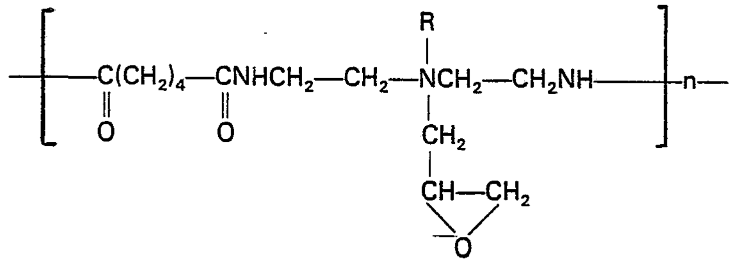

wherein R is methyl or hydrogen.

The improved charge functionality demonstrated by the most preferred resin may be related to the nitrogen atoms partial removal from the polymer chain and cônsequent surface accessibility.

A secondary charge modifying agent may be used to enhance the cationic charge of the primary charge modifying agent and/or enhance the bonding of the primary charge modifying agent to the microporous surface and/or itself.

The secondary charge modifying agent used in this invention is selected from the group consisting of

(i) aliphatic amines which are polyamines having at least one primary amine or at least two secondary amines; and
(ii) aliphatic amines having at least one secondary amine and a carboxyl or hydroxyl substituent.

Preferably, the secondary charge modifying agent is a polyamine having the formula:

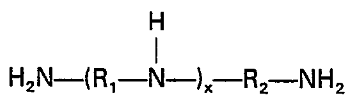

wherein $R_1$ and $R_2$ are alkyl of 1 to 4 carbon atoms and x is an integer from 0 to 4. Preferably, $R_1$ and $R_2$ are both ethyl.

Preferred polyamines are:

| | |
|---|---|
| Ethylene diamine | $H_2N—(CH_2)_2—NH_2—NH_2$ |
| Diethylenetriamine | $H_2N—(CH_2)_2—NH—(CH_2)_2—NH_2$ |
| Triethylenetetramine | $H_2N—(CH_2—CH_2—NH)_2—CH_2—CH_2—NH_2$ |
| Tetraethylenepentamine | $H_2N—(CH_2—CH_2—NH)_3—CH_2—CH_2—NH_2$ |

The highly preferred polyamine is tetraethylene pentamine.

Alternatively, aliphatic amines used in this invention may have at least one secondary amine and a carboxyl or hydroxyl substituent. Exemplary of such aliphatic amines are gamma-amino-butyric acid ($H_2NCH_2CH_2CH_2COOH$) and 2-aminoethanol ($H_2NCH_2CH_2OH$).

The secondary charge modifying agent is bonded to the microporous membrane by bonding to a portion of the epoxide substituents of the polymeric primary charge modifying agent.

The amount of primary and secondary cationic charge modifying agent utilized is an amount sufficient to enhance the electropositive capture potential of the microporous membrane. Such an amount is highly dependent on the specific charge modifying agents utilized.

A preferred embodiment of the process of the invention comprises (a) contacting the membrane with an aqueous solution of the primary cationic charge modifying agent and (b) contacting the membrane with an aqueous solution of the secondary charge modifying agent. The contacting steps may be performed in any order, i.e. step (a) prior to step (b) or vice versa. It is preferred, however, for optimum (minimum) extractables to first contact the membrane with an aqueous solution of the primary cationic charge modifying agent and then subsequently contact the so treated membrane with the aqueous solution of the secondary charge modifying agent.

In order to provide the charge modifying amount of cationic charge modifying agent to the membrane, it is preferred that the aqueous solution of primary charge modifying agent that the membrane is contacted with contain at least about 1.0% charge modifying agent, by weight of the solution. The maximum amount of charge modifying agent in the aqueous solution is limited by economic and solubility limitations. For example, an excess of primary charge modifying agent which is not bonded to the microporous membrane will not be economically utilized and will constitute an undesirable extractive from the membrane. It has been found that the amount of charge modifying agent in the aqueous solution should probably not exceed about 5% by weight of the solution.

The amount of secondary charge modifying agent used in the aqueous solution is highly

dependent on the specific secondary charge modifying agent and the amount and type primary charge modifying agent used, and the cross-linking mechanism between these compounds to provide the bonding of such charge modifying agent to the microporous membrane. For general guidance however, it has been found that a weight ratio of primary to secondary charge modifying agent of from 2:1 to 500:1, preferably from 25:1 to 75:1 in the aqueous solutions contacted with the membrane, is generally sufficient to provide the bonding of the cationic charge modifying agents to the membrane. It has been found that if the aqueous solution containing the secondary charge modifying agent contains at least .01% charge modifying agent by weight of the solution, up to a maximum of .5% weight of the solution when used in conjunction with the aforementioned aqueous solution of primary charge modifying agent, that adequate bonding of the charge modifying agents to the microporous membrane is obtained.

Both the charge modifying agents may be contacted with the membrane by dipping the membrane in the aqueous solutions of these compounds for a period of time sufficient to effect the desired degree of pick-up. Alternatively, the charge modifying agents may be applied by spraying or contacting a wick or roll along the surface of the microporous membrane.

Preferably, the hydrophilic membranes, in particular the nylon membrane are surface activated, as with a caustic or basic wash, i.e. above pH 7, to provide a surface affording an enhanced amino-functionality as measured, for example, by ninhydrin colorimetery. This provides improved responsiveness to charge modification.

In an embodiment of this invention the membrane is surface activated before contacting charge modifying agents with the membrane. In the simplest form, the preferred nylon membrane is treated with caustic to provide additional amino groups for cross-linking. The same or enhanced effect is preferably achieved by providing an alkaline bath for application of at least one of the charge modifying agents, preferably the primary charge modifying agents. The bath is preferably at a pH of 9 to 11.

It is believed that the foregoing caustic surface treatment provides surface functionality free of blocking salts which are produced, for example, by the reaction of the acids used in producing the hydrophilic membrane with the functional substituents, e.g. amino. Thus the caustic treated nylon membrane evidences an enhanced ninhydrin test response for free amino functionality.

In acid solution (formic acid) the polymer acts as a polyelectrolyte, i.e. in formic acid solution the polymer chain acquires a charge. This charge results from the reaction of formic acid with the polymer. Specifically a proton ($H^+$) from the acid attaches to the nitrogen atom in the polymer chain yielding the following type charged structure:

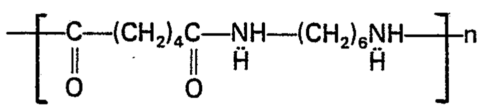

Since electrical neutrality is always maintained, each charge site

(i.e. 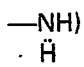)

is associated with the corresponding formate anion ($HCOO^-$). After the polymer is prepared and dried the salt structure is probably still present. It is theorized that these salts are neutralized by caustic or basic treatment used in this invention. It is believed that the high pH tends to activate the epoxide functionally on the primary charge modifying agent and break the formic acid/nylon amino bonds on the nylon surface and render the carboxylic acid groups on the nylon surface more reactive.

The activated epoxide groups of the primary charge modifying agent then react with both nylon functional groups, i.e., amino and carboxylic.

Although applicants do not wish to be bound by the following theory, it is believed that in bonding the primary charge modifying agent to the microporous membrane the epoxide groups on the primary charge modifying agent enter into addition type reactions with the hydroxyl, carboxyl and primary and secondary amines, which are on the hydrophilic microporous membrane and the primary and secondary cationic charge modifying agents. These reactions may be represented as follows:

Hydroxyl

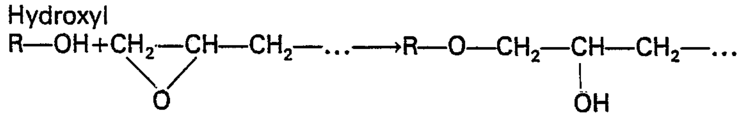

Carboxyl

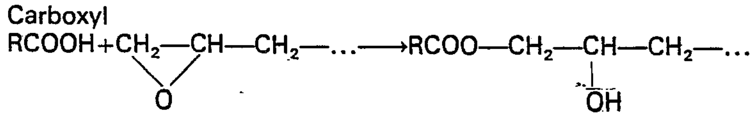

Primary Amine

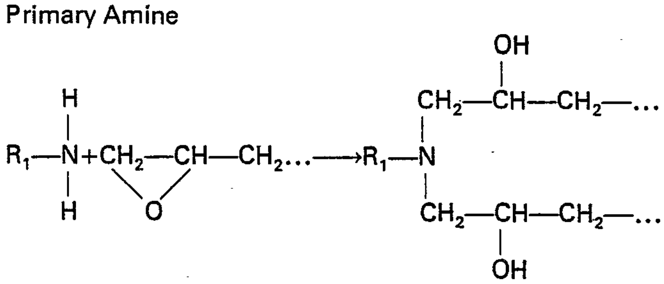

Secondary Amine

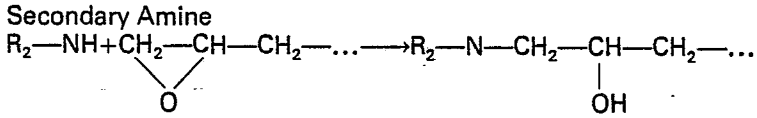

The epoxide substituent on the primary charge modifying agent thus serves several functions:

1. The epoxide cross-links the primary amine groups on the hydrophilic membrane to the primary charge modifying agent and the primary and/or secondary amine groups on the secondary charge modifying agent;

2. The epoxide cross-links the carboxyl groups on the hydrophilic microporous membrane to the primary charge modifying agent and the primary and/or secondary amine groups of the secondary charge modifying agent;

3. The epoxide cross-links the primary and/or secondary amines of the secondary charge modifying agent to each other.

It is theorized that a polymeric primary charge modifying agent offering greater than three epoxide groups per monomer offers no benefit, and in fact may limit the coupling reactions of the primary charge modifying agent by steric hindrance. Additionally, the presence of unreacted epoxide groups on the charge modified microporous membrane may be undesirable in the finished charge modified membrane.

The amines used as secondary charge modifying agents in this invention are selected in the view of the following theoretical considerations. Amines are classified as primary, secondary or tertiary.

Epoxide groups will react with primary and secondary amine groups through the free hydrogens. An epoxide group will not react with a tertiary amine group since there are no free hydrogens.

Amine groups of all three classes, i.e. primary, secondary or tertiary are capable of forming hydrogen bonds with water. As a result, amines of relatively low molecular weight, i.e. short carbon chain length are quite soluble in water, with border line solubility in water occurring at about 6 carbon atoms per amine group. In the preferred embodiment of this invention it is highly desirable that both cationic charge modifying agents be soluble in water to provide the desired environment for production, i.e. elimination of fumes, toxicity, etc.

The amines are converted into their salts, i.e. charged form, by hydrogen ions and are liberated from their salts by hydroxide ions:

| | |
|---|---|
| $R_1NH_2+H_3O^+$<br>Stronger<br>Base | $R_1NH_3^++H_2O$<br>Weaker<br>Base |
| $R_1NH_3^++OH^-$<br>Stronger<br>Base | $R_1NH_2+H_2O$<br>Weaker<br>Base |

It is this latter characteristic, that produces an undesirable reduction in positive surface charge on the microporous membrane (as measured by electrophoretic mobility or streaming potential), and the corresponding reduction in adsorptive capacity for anionic contaminants that has been noted when amine charge modified filter media is tested over a series of increasing pHs. It would therefore appear

that the more basic the amine charge modifying agent, the higher is the charge modification and adsorptive capacity for contaminants that a filter media, e.g. membrane, will exhibit at a given pH.

Basicity of an amine is defined by measuring the extent to which the amine can accept hydrogen ions from water, with the equilibrium constant for this reaction being the basicity constant Kb.

To select from among the aliphatic amines a preferred embodiment on a theoretical basis becomes somewhat more complicated due to the fact that one is concerned with the basicity of the amine bonded through the epoxide to the microporous membrane.

From certain tests performed however, it appears that increased cross-linking with the epoxide substituent increases basicity and filtration effectiveness and thus appears to depend upon the extent to which the primary and secondary amines originally present in, for example, the tetraethylene pentamine, are converted to tertiary amines via the reaction with the epoxide.

Preferably, between each contacting step of the process for producing the membrane, the membrane is drained for a period of time sufficient to remove most of the water and chemical compound not absorbed or adsorbed onto the surface of the membrane. Optionally, the membrane may be transferred directly from the first contacting step to the second contacting step, although this is less preferred. The intermediate treatment may also be a restrained drying step.

After the microporous membrane has been contacted with the aqueous solutions, it is then dried and cured, preferably in a restrained condition to prevent shrinkage.

Drying of the membrane is preferred under restraint. Generally, any suitable restraining technique may be used while drying, such as winding the membrane tightly about a drying surface, e.g. a drum. Bi-axial control is preferred and tensioning the membrane on a stretching frame is considered the most preferred. Preferably, the restraint imposed affects no reduction in dimensions.

Final drying and curing temperatures should be to dry and cure the treated membranes, preferably from about 120°C to 140°C for minimization of drying times without embrittlement or other detrimental affects to the membrane.

The completed membrane may be rolled and stored for use under ambient conditions. It will be understood that the treated membrane may be supplied in any of the usual commercial forms, for example, as discs or pleated cartridges.

The present invention provides an integral, coherent microporous membrane of retained internal pore geometry. The charge modified membrane has an improved effective filtration rating relative to the untreated micro-reticulate polymer structure. Such improvement is brought about by charge sites or regions which are effective during filtration to enhance filtration performance through electrokinetic effects.

The resulting membrane offers improved micrometer rating at equivalent flow and capacity with retention of membrane structure, yet without evidence of significant resin extractables. In effect the effective micrometer rating for contaminant particles is less than the effective micrometer rating of the microreticulate membrane structure. By the use of the term "effective micrometer rating for contaminant particles", it is meant the actual size of the particles that the membrane will quantitatively remove from the fluid being filtered. By the use of the term "effective micrometer rating of the microreticulate membrane structure" it is meant the size of the particulate that would pass through the membrane if all adsorptive effects of the membrane were eliminated.

For so-called sterile filtrations involving biological liquids, the filter is sanitized or sterilized by autoclaving or hot water flushing. Accordingly, the charge modified membrane must be resistant to this type treatment, and must retain its integrity in use. Any modification to the filter structure, especially brought about by chemical agents which may be unstable under conditions of treatment and use, must be scrutinized with care to minimize the prospect of extractables contaminating the filtrate, interfering with analyses and potentially introducing harmful toxins to a patient. Specifically, any such filter must meet the test standards in the industry, e.g. ASTM D 3861—79 (incorporated herein by reference), and generally prove less than 5 mg. of extractables in 250 ml solvent (water at 80°C.; 35% ethanol at room temperature) for a 293 mm diameter disc.

While the primary and secondary charge modifying agents afford cross-linking functionality and cross-linking through such functionality with the base membrane, the improved accessibility of the reactive groups on the membrane brought about by the activation treatment, i.e. caustic wash, enhances interreaction of the agents with the membrane, and insures extremely low extraction levels. Where extraction levels are of major concern, this represents the most preferred embodiment.

The resulting charge modified membrane is characterized by retention of internal microstructure, thus offering essentially the same flow characteristics as the untreated membrane. For example, a 0.22 micrometer rated nylon membrane is essentially absolute for .109 test beads.

The charge modified membrane additionally is easy to handle and readily formed into convoluted structures, e.g. pleated configurations. By reason of its retained flow characteristics, it may be employed directly in existing installations, without pumping modifications. These favorable properties are secured without sacrifice to other characteristics. The membrane may also be constructed to meet or exceed extractable requirements.

Biological liquids as that term is employed in the specification and claims, is a liquid system which is derived from or amenable to use with living organisms. Such liquids are ordinarily handled and

processed under sanitary or sterile conditions and therefore require sanitized or sterilized media for filtration. Included within such term are isotonic solutions for intermuscular (im) or intravenous (iv) administration, solutions designed for administration per os, as well as solutions for topical use, biological wastes or other biological fluids which may comprise filterable bodies such as impurities, e.g., bacteria, viruses or pyrogens which are desirably isolated or separated for examination or disposal by immobilization or fixation upon or entrapment within filter media.

Filter membranes in accordance with this invention may be employed alone or in combination with other filter media to treat pharmaceuticals such as antibiotics, saline, solutions, dextrose solutions, vaccines, blood plasma, serums, sterile water or eye washes; beverages, such as cordials, gin, vodka, beer, scotch, whisky, sweet and dry wines, champagne or brandy; cosmetics such as mouthwash, perfume, shampoo, hair tonic face cream or shaving lotion; food products such as vinegar, vegetable oils; chemicals such as antiseptics, insecticides, photographic solutions, electroplating solutions, cleaning compounds, solvent purification and lubrication oils; and the like for retention of submicronic particles, removal of bacterial contaminants and resolution of colloidal hazes. Illustratively, in hospital usage, membrane filters are employed to concentrate abnormal exfoliated cells from a vaginal rinse, to isolate blood parasites from peripheral blood, or bacteria from serum or leucocytes and casts from urine.

In the case of preparation for use in sterile filtration, the membrane is thermally sanitized or sterilized as by treatment in an autoclave at 121°C. under an overpressure of 1 bar for 1 hour, or hot water flushing at 29°C for 1 hour.

Examples

The following are the measurement and test procedures utilized in all the Examples.

Thickness

The dry membrane thickness was measured with a 1.27 cm diameter platen dial indicator thickness gauge. Gauge accuracy was better than 1,27 μm.

Initial bubble point (IBP) and foam-all-over-point (FAOP) tests

A 47 mm diameter disc of the membrane sample is placed in a special test holder which seals the edge of the disc. Above the membrane and directly in contact with its upper face, is a perforated stainless steel support screen which prevents the membrane from deforming or rupturing when air pressure is applied to its bottom face. Above the membrane and support screen, the holder provides a inch deep cavity into which distilled water is introduced. A regulated air pressure is increased until a first stream of air bubbles is emitted by the water wetted membrane into the quiescent pool of water. The air pressure at which this first stream of air bubbles is emitted is called the Initial Bubble Point (IBP) of the largest pore in that membrane sample—see ASTM D-2499-66T.

Once the Initial Bubble Point pressure has been determined and recorded, the air pressure is further increased until the air flow thru the wetted membrane sample, as measured by a flow meter in the line between the regulator and the sample holder, reaches 100 cc/min. The air pressure at this flow rate, is called the Foam-All-Over-Point (FAOP), and is directly proportional to the mean pore diameter of the sample membrane. In this series of tests, these two parameters (IBP and FAOP) are used to determine if any change has occurred in the maximum or mean pore size of the membrane sample as a result of the charge modifying process utilized.

Flow rate test

A 47 mm diameter disc of the membrane sample is placed in a test housing which allows pressurized water to flow thru the membrane. Prefiltered water is passed thru the membrane sample at a pressure differential of 0,35 bar. A graduate cylinder is used to measure the volume of water passed by the membrane sample in a one minute period. In this series of tests this parameter is used in conjunction with the IBP and FAOP to determine if any reduction in pore size or pore blockage has occurred as a result of the charge modifying process utilized.

Dye adsorption test

A 47 mm diameter disc of the membrane sample is placed in a test housing which allows pressurized water flow thru the membrane. The challenge solution consists of distilled water at a pH of 7.0, and Metanil Yellow dye. The dye inlet concentration is adjusted to produce a 76 percent transmittance at a wavelength of 430 nm.

By means of a peristaltic pump the challenge solution is flowed thru the membrane sample at a flow rate of 28 ml/min. The transmittance of the effluent is measured by passing it thru a constant flow cell in the aforementioned Spectrophotometer. The effluent transmittance and pressure drop across the membrane is measured and recorded as a function of time. The test is terminated when the effluent transmittance increases to 85 percent of the inlet transmittance. In this series of tests, the length of time that it takes to reach the 85 percent transmittance in the effluent is called the "breakthru" time. Since the Metanil Yellow is a low molecular weight anionic dye incapable of being mechanically

removed (filtered) by the membrane, this breakthru time is proportional to the cationic adsorptive capacity of the membrane sample. This test is therefore used to determine the effectiveness of the charge modification technique.

Extractables (ASTM D-3861-79)

Extractables were determined by ASTM D-3861-79. The quantity of water-soluble extractables present in the membrane filters were determined by immersing the preweighed membrane in boiling reagent grade water for an extended time and then drying and reweighing the membrane. A control membrane was employed to eliminate weighing errors caused by balance changes or changing moisture content of the membrane in the weighing procedures. Weight changes of the control membrane were applied as a correction factor to the weight change of the test membrane filters.

Example I

A. Preparation of microporous membrane

A representative nylon 66 membrane of 0.22 micrometer nominal rating, having a nominal surface area of about 13 $m^2/g$, an Initial Bubble Point of about 3,3 bar, a Foam-All-Over-Point of about 3,6 bar was prepared by the method of *Marinaccio et al,* U.S. Patent 3,876,738, utilizing a dope composition of 16 percent by weight nylon 66 7.1% methanol and 76.9% formic acid, a quench bath composition of 25% methanol, 75% water by volume (regenerated as required by the method of *Knight et al,* U.S. Patent 3,928,517) a casting speed of 61 cm/min, and a quench bath temperature of 20°C. The membrane was cast just under the surface of the quench bath by application to a casting drum rotating in the bath 0,23 to 0,25 mm as cast wet, to obtain 0,11 to 0,14 mm dry) and allowed to separate from the drum about 90° of arc from the point of application, the self-supporting membrane forming a shallow catenary to takeup. A portion of the uniform opaque film was dried (in restrained condition to resist shrinkage) in a forced air oven at 80—90°C. for 30 minutes.

B. Preparation of charge modified microporous membrane

1. Membrane samples (dried and undried) were dipped in a bath of polyamido-polyamine epichlorohydrin resin (4% solids by weight), and allowed to attain adsorption equilibrium. The treated membrane samples were washed to remove excess resin and dried in restrained condition on a drum at a temperature of 110°C. for a period of about 3 minutes.

The treated membrane samples were compared for flow and bubble point characteristics as follows, and found to be essentially identical for treated and untreated samples, evidencing retention of pore and surface geometry. The results are set forth in Table I.

TABLE I

| | Control (no treatment) | Undried membrane | Dried membrane |
|---|---|---|---|
| Thickness (mm) | 0,108 | 0,116 | 0,122 |
| Initial Bubble Point (bar) | 3,01 | 3,08 | 3,08 |
| Foam-All-Over-Point (bar) | 3,80 | 3,73 | 3,47 |
| Thickness Normalized Flow Rate ($cm^3$ $\mu m$/min. $cm^2$ bar) | 2620 | 2650 | 2580 |
| BET, $N_2$ adsorption | 13.12 | — | 13.58 |

Thus, in terms of the morphological and hydrodynamic parameters that control mechanical sieving, the filtration characteristics of the treated membranes were essentially identical with the untreated nylon membrane.

2. Similar characterizations were conducted on another membrane sample, similarly prepared, but treated with 2% of a free radical polymerized resin based upon diallyl nitrogen-containing materials, reacted with epichlorohydrin in a bath adjusted to pH 10.5, overcoated with 0.1% tetraethylene pentamine, dried, cured, washed and redried. The results are set forth in Table II.

TABLE II

| | Control (no treatment) | Dried membrane |
|---|---|---|
| Tensile Strength (bar) | | |
| Wet | 36,5 | 44,8 |
| Dry | 59,3 | 66,2 |
| Elongation (%) | | |
| wet | 140 | 100 |
| Dry | 95 | 40 |

Surface area of the treated and untreated membranes remained essentially unchanged; tensile strength increased with treatment with some loss in elongation. The treated sheet was more flexible; creasing of the untreated sheet resulted in cracking and splitting.

C. Filtration tests

The as in example I.B.1. treated membrane samples (Example I.B.1.) were subjected to the filtration tests indicated below:

Pyrogen removal

Purified E. coli endotoxin was added to a 0.9% NaCl solution, pH 6.7 and passed through test filters mounted in a 25 mm diameter stainless steel holder. Inlet and effluent endotoxin levels were determined by standard L.A.L. analysis.

Results are set forth in Table III.

TABLE III

| Filter | Inlet endotoxin level (pg/ml) | Effluent endotoxin level (pg/ml) 10 ml. | 50 ml. | 100 ml. |
|---|---|---|---|---|
| Dried, treated Membrane | 15000 | 1000 | 1000 | 1000 |
| Control-untreated | 15000 | 10000 | 10000 | 10000 |

(Pg is "picogram")

Virus removal

MS-2 bacteriophage was added to Houston, Texas (U.S.A) tap water to produce a concentration of $3.4 \times 10^5$ PFU/ml PFU is "Plaque Forming Unit") and 10 ml was passed through each of the test filters mounted in a 25 mm diameter stainless steel holder. Effluents were analyzed for viral content by standard techniques. Results are set forth in Table IV:

TABLE IV

| Filter | Total viral PFU in filtrate | Virus removal efficiency (%) |
|---|---|---|
| Dried, treated Membrane | 100 | 99.997 |
| Control-untreated | 250000 | 26.4 |

Monodisperse latex filtration

The test filters were challenged with a 10 NTU dispersion (NTU is "nephlometric turbidity units") of 0.109 micrometer monodisperse latex (MDL) particles at a flow rate of 0,002 $lm^{-1}/cm^2$), pH 7.0, R=21000-ohm-cm. Effluent turbidities (NTU) were monitored and filtration efficiencies were calculated from equilibrium effluent turbidities. Results are set forth in Table V.

TABLE V

| Filter | MDL Removal efficiency |
|---|---|
| Undried, treated | 97.3% |
| Control-untreated | 10% |

Dye removal efficiency

The test filters were challenged with a solution of blue food coloring dye. The solution had a light transmittance of 62.5% at 628 nm. The light transmittance of the effluent was monitored and removal efficiencies determined (based on distilled water light transmittance=100%). Results are set forth in Table VI.

TABLE VI

| | Throughput (litres) to 90% transmittance |
|---|---|
| Undried, treated | 1.99 |
| Dried, treated | 1.76 |
| Control-untreated | 0 |

Example II

In a series of related runs employing dried treated membrane as described in Example I.B. 1. the amount of charge modifying resin in the treatment bath was modified from 1 to 5% by weight, the drying time for the treated membrane was altered from 15 to 30 minutes, the adsorption equilibration time was modified from 1 to 5 minutes, and pH was shifted from 4 to 9 and the factorial experiment analyzed for responses.

The results showed that as concentration of charge modifying resin increased, flow rate was adversely affected with increased pressure drop, while enhancing filtration performance as measured by dye retention time. This suggests some clogging of pores by resin at the higher levels. Higher extractions suggested the presence of excess resin at the 5% wgt. level.

Reduced extractables were evidenced for longer drying times and flow rate improved, with little change in pressure drop or filtration performance.

Longer equilibration time correlated favorably with reduced extractables, as well as improving flow rate at essentially equivalent pressure drop and filtration performance.

Alkaline pH conditions reduced extractables markedly, and the treated membrane showed improved flow rate and filtration at essentially equivalent pressure drop.

Example III

A. A non-treated microporous nylon membrane prepared in accordance with Example IA was soaked in ninhydrin (1,2,3-trione-2-hydrate, 0.4% aqueous) overnight, and examined for color development. The membrane had turned a light purple, evidencing a good level of amino functionality (positive test ranges from blue to purple). A competitive nylon membrane (non-charge modified) evidenced only a very pale hint of lavender tint in the same test.

The same membrane of Example IA was washed with a saline solution and soaked in saturated sodium tetraborate. The membrane tested positive for amino-functionality (yellow to orange color) with the addition of 3—4 drops of 2,4,6-trinitro benzene sulfonic acid (3% aqueous). The solution turning a muted yellow within 30 seconds. The competitive membrane showed no color response.

B. The untreated membrane of Example IA was treated with caustic (1 minute immersion in aqueous sodium hydroxide pH 10.5) washed, dried and soaked in ninhydrin overnight. The membrane was deeper purple than the untreated indicating for this test increased amino functionality. The competitive nylon membrane, treated in the same manner, evidenced a light purple coloration, also indicating an increased amino functionality compared to the untreated membrane.

C. The membrane of Example IA treated with caustic was treated with a resin as used in example I.B.2., dried, cured, washed and redried. Extractables testing (ASTM D-3861-79) evidenced reduced extractables as compared to a membrane treated with the same resin, but without caustic pretreatment.

Example IV

A. Microporous nylon membrane prepared in accordance with Example IA was treated with a as in example I.B.2. charge modifying agent (pH of bath adjusted to 10 with sodium hydroxide) and, where indicated with a polyamine secondary charge modifying agent.

Flow characteristics of the respective membranes showed little or no differentiation, as set forth in Table VII:

TABLE VII

| Treatment | | | Membrane characteristics | | |
|---|---|---|---|---|---|
| Charge modifier | | | | | Thickness |
| Primary | Secondary | Sequence | IBP[1] (bar) | FAOP[2] (bar) | normalized flow[3] ($cm^3$. $\mu$m/min. cm. bar) |
| None | None | — | 3,02 | 3,59 | 2490 |
| 2.0% | 0.133% Anquamine[4] | Primary First | 3,27 | 3,62 | 2490 |
| 2.0% | 0.133% Anquamine | Secondary First | 3,13 | 3,48 | 2380 |
| 2.0% | 0.133% Anquamine[4] | Mixed | 3,36 | 3,54 | 2270 |
| 2.0% | None | — | 3,22 | 3,52 | 2670 |
| 2.0% | 0.03% Tetraethylene Pentamine | Primary First | 3,52 | 3,73 | 2180 |

1. Initial Bubble Point
2. Foam All Over Point
3. $$\frac{\text{Flow rate (cc. mm}^{-1}\text{)} \times \text{thickness (mil)}}{(\Delta P \text{ (psid)} \times \text{Area (cm}^2\text{))}}$$
an empirically derived relation to normalize data for thickness variations.
4. Anquamine—100, a low molecular weight (under 10,000) cationic polyamide adduct evidencing secondary amine functionality by comparative UV spectroanalysis.

B. Filtration performance of the treated membranes was determined in a membrane life test, employing 0.109 monodisperse polystyrene latex from Dow Diagnostic (MDL) mixed with double glass distilled water to produce a dispersion with a turbidity of 10 NTU as measured on a Hach Model 2100A Turbidimeter. The pH of the test dispersion was 7.0.

Flow of contaminated dispersion at a rate of 14 ml/min. was established through a 47 mm. membrane test disc, and differential pressure and turbidity of effluent monitored. The test was deemed complete at 0,35 bar difference attainment of 5 NTU contamination in the effluent. The performance is recorded as elapsed time. Results are set forth in Table VIII, as follows:

TABLE VIII

| Treatment | | | MDL Test performance | | | | |
|---|---|---|---|---|---|---|---|
| Charge modifier | | | | | | | |
| Primary | Secondary | Sequence | Init. ΔP (bar) | Max. NTU | Avg. NTU | Final ΔP (bar) | Time (min.) |
| 2.0% | 0.133% Anquamine | Primary First | 0,041 | 5.1 | 1.35 | 0,10 | 60 |
| 2.0% | 0.133% | Secondary First | 0,053 | 5.1 | 1.34 | 0,12 | 60 |
| 2.0 | 0.133% | Mixed | 0,038 | 2.4 | 0.96 | 0,18 | 80 |
| 2.0% | None | Primary Only | 0,041 | 7.0 | 4.7 | 0,05 | 10 |
| 2.0% | 0.03 Tetraethylene pentamine | Primary First | 0,035 | 0.36 | 0.17 | 3,0 | 107 |

C. Filtration performance was also compared between samples of membrane treated with a bath containing either 4.24 wgt.% resin as in I.B.2 or 2.45% resin as in I.B.2., both followed by a bath of 0.03% tetraethylene pentamine. The two membranes performed equivalently in membrane life test with .109 MDL contaminant challenge. The latter membrane, which contained less primary charge modifier, performed better in dye retention tests.

D. The membranes were tested for extractables, in accordance with ASTM D-3861-79. The results are set forth in Table IX.

TABLE IX

| Treatment Charge modifier Primary | Secondary | Sequence | Extractions pursuant to ASTM D-3861-79 |
|---|---|---|---|
| 2.0% 4308 | 0.133% Anquamine | Primary First | 0.0 mg |
| 2.0 4308 | 0.133% Anquamine | Secondary First | 3.5 |
| 2.0% 4308 | 0.133% Anquamine | Mixed | 4.7 |
| 2.0% 4308 | None | Primary Only | 0.0 mg |
| 2.0% 4308 | 0.03% tetraethylene pentamine | Primary First | 0.0 mg |

Example V

In order to compare performance of different primary charge modifiers, particularly polyamide-polyamine epichlorohydrin resin candidates and to optimize application levels and pH conditions, the following tests were conducted, utilizing resins as in I.B.2. Polycup 172, pH 4.7 as supplied) and Polycup 2002 (27% solids, pH 3.0 as supplied). The results are set forth in Table X:

TABLE X

| Primary charge modifier | Bath pH | IBP | FAOP | Flow rate ml/min. (0,7 bar) | Initial ΔP (bar) | Final ΔP (bar) | Dye ret.[1] minutes |
|---|---|---|---|---|---|---|---|
| 1% R4308 | 10.3 | 47 | 50 | 162 | 0,128 | 0,33 | 68 |
| 2% R4308 | 10.3 | 45 | 49 | 162 | 0,124 | 0,33 | 100+ |
| 3% R4308 | 10.3 | 44 | 51 | 184 | — | 1,05 | 100+ |
| 1% 172 | 11.0 | 46 | 50 | 171 | 1,29 | 0,45 | 30 |
| 1% 172 | 4.9 | 47 | 50 | 162 | 0,145 | 0,15 | 15 |
| 2% 172 | 11.0 | 47 | 52 | 288 | — | 0,124 | 34 |
| 2% R4308 | 11.0 | 47 | 52 | 187 | — | 0,36 | 60+ |
| 2% 2002 | 11.0 | 49 | 52 | 183 | — | 0,17 | 35+ |
| Control | — | 46 | 50 | 267 | 0,130 | 0,2 | 5 |

[1]Metanil yellow at 1 ppm. The test was maintained until dye breakthrough, measured as 75% transmittance at 430 nanometers.

Example VII

B

Figure 1 is a graphical representation of Filtration Efficiency (%) versus the Specific Volume Filtered (ml/$cm^2$) for a charge modified membrane of this invention ("Treated Membrane") and a noncharge modified membrane ("Untreated Membrane"). Both membranes had a .2 micrometer rating prior to treatment. The charge modified membrane was produced by treating a nylon microporous membrane with a bath of 2% by weight of Hercules R4308 resin at a pH of 10.5 followed by a bath of .1% tetraethylene pentamine. Both the treated and untreated membranes were challenged with a .109 MDL contaminant at 0,002 $lm^{-1}/cm^2$.

B

Figures 2 and 3 are Scanning Electron Microscope (SEM) photos of a charge modified membrane produced and challenged as described in this Example VII A with a .14 micron MDL beads (Figure 2—7000 x; Figure 3—14,000 x).

Example VIII

A series of tests were conducted to investigate the characteristics of the charge modified membrane of this invention and process for producing such membrane.

The membrane used for this test series was a single sheet of unmodified, double layer, 0.2 $\mu$m nylon membrane produced pursuant to Example IA. Each Sample group consisted of three (3) adjacent 21.6 cm×27.9 cm sheets, i.e. sheets "A", "B" and "C". The "A" sheet was left untreated and was used to provide data for "unmodified" or "before treatment" membrane. The "B" and "C" groups were subjected to the treatment modes given in Table XI The "A" and "B" groups were subjected to the following measurements and tests:

Thickness—4 samples, 47 mm disc

Initial Bubble Point (IBP) and Foam-All-Over-Point (FAOP)—4 samples, 47 mm disc

Flow—4 samples, 47 mm disc

Dye Adsorption—2 samples, 47 mm disc

Extraction—10 samples, 293 mm disc per ASTM D-3861-79

The "C" group was retained for future testing.

The results obtained from the testing of the various treatment modes (see Table XI) are statistically summarized in Table XII.

TABLE XI
Treatment modes

| No. | Pretreatment | Charge modifications | Post treatment |
|---|---|---|---|
| 1 | None | Parez 607 Colloid—2 wt.% solution—pH as diluted | Drain and stretch dry |
| 2 | None | 1884 Resin—2 wt.% solution—pH as diluted | Drain and stretch dry |
| 3 | None | Wesol PA—2wt.% solution—pH as diluted | Drain and stretch dry |
| 4 | None | 4308 Resin—2 wt.% solution—pH as diluted | Drain and stretch dry |
| 5 | Soak membrane in dilute NaOH solution (pH 10.5) for two minutes. Drain and oven dry. Wash in distilled water | Parez 607 Colloid—2 wt.% solution—pH as diluted | Drain and stretch dry |
| 6 | | 1884 Resin—2 wt.% solution—pH as diluted | Drain and stretch dry |
| 7 | | Wesol PA—2 wt.% solution—pH as diluted | Drain and stretch dry |
| 8 | | 4308 Resin—2 wt.% solution—pH diluted | Drain and stretch dry |
| 9 | None | 4308 Resin—2 wt.% solution—pH diluted | Drain and stretch dry |
| 10 | None | 4308 Resin—2 wt.% solution—pH adjusted to 10.5 | 0.03 wt% solution Pentamine Drain and stretch dry |
| 11 | 0.03 wt.% solution Pentamine | 4308 Resin—2 wt.% solution—pH as diluted | Drain and stretch dry |
| 12 | 0.03 wt.% solution (1.4 butanediol) | 1884 Resin—2 wt.% solution—pH as diluted | Drain and stretch dry |
| 13 | 0.03 wt.% solution (1.4 butanediol) | 4308 Resin—2 wt.% solution as diluted | Drain and stretch dry |
| 14 | 0.03 wt.% solution DGE (1.4 butanediol) | 1884 Resin—2 wt.% solution—pH as diluted | Drain and stretch dry |
| 15 | None | None—soak in water | Drain and stretch dry |

Parez 607—melamine formaldehyde cationic colloid
1884 Resin—Hercules 1884—polyamido-polyamine epichlorohydrin
Wesol PA—cationic colloidal silica
4308—Hercules Inc. R4308 polyamido-polyamine epichlorohydrin resin.

TABLE XII (1 of 4)
Summary of test results

| Sample No. | Thickness ( ) BT | AT | AE | IBP ( ) BT | AT | AE | FAOP ( ) BT | AT | AE | Flow (ml/min) BT | AT | AE |
|---|---|---|---|---|---|---|---|---|---|---|---|---|
| 1. | 118 | 116 | 119 | 3,11 | 3,12 | 3,08 | 3,38 | 3,34 | 3,37 | 86.25 | 90.70 | 87.0 |
| 2. | 121 | 121 | 122 | 3,08 | 3,01 | 3,07 | 3,43 | 3,31 | 3,36 | 87.25 | 76.50 | 94.0 |
| 3. | 121 | 118 | 118 | 3,0 | 3,16 | 3,07 | 3,43 | 3,45 | 3,33 | 85.00 | 84.00 | 94.0 |
| 4. | 116 | 123 | 123 | 3,02 | 3,16 | 3,17 | 3,47 | 3,47 | 3,46 | 81.75 | 2.62 | 86.00 |
| 5. | 118 | 119 | 119 | 3,08 | 3,02 | 2,94 | 3,35 | 3,38 | 3,46 | 88.25 | 93.25 | 83.00 |
| 6. | 119 | 124 | 122 | 3,10 | 3,20 | 3,11 | 3,39 | 3,55 | 3,48 | 87.50 | 49.00 | 85.00 |
| 7. | 122 | 119 | 123 | 3,03 | 3,20 | 3,05 | 3,35 | 3,48 | 3,38 | 86.25 | 83.25 | 91.00 |

BT—Before Treatment
AT—After Treatment
AE—After Extraction

TABLE XII (2 of 4)
Summary of test results

| Sample | Dye adsorption time to B.T. (min) BT | AT | AE | ΔP Characteristic* BT | AT | AE | Extraction (mg/293 mm disc) |
|---|---|---|---|---|---|---|---|
| 1 | 12.36 | 22.78 | 14.85 | 0,138 | 0,138 | 0,138 | 27.6 |
| | | | | 0,152 | 0,145 | 0,138 | |
| 2 | 12.39 | 34.67 | 23.86 | 0,138 | 0,152 | 0,138 | 116.1 |
| | | | | 0,138 | 0,152 | 0,138 | |
| 3 | 11.01 | 16.30 | 6.81 | 0,138 | 2,0 | 2,0 | |
| | | | | 2,0 | 2,0 | 2,0 | |
| 4 | 10.94 | — | 90.79 | 2,0 | >2,07 | 0,145 | 118.5 |
| | | | | | — | 0,241 | |
| 5 | 6.58 | 22.51 | 15.63 | 0,138 | 0,124 | 0,152 | 24.3 |
| | | | | | 0,158 | | |
| 6 | 5.96 | 37.54 | 21.06 | 0,138 | 0,158 | 0,152 | 126.4 |
| | | | | | 0,179 | | |
| 7 | 6.47 | 12.86 | 8.40 | | | | 43.9 |

BT—Before Treatment
AT—After Treatment
AE—After Extraction
B.T.—Breakthrough
*Upper Numbers are start of test and lower numbers are end of test (bar).

TABLE XII (3 of 4)
Summary of test results

| Sample No. | Thickness (μm) BT | AT | AE | IBP (bar) BT | AT | AE | FAOP (bar) BT | AT | AE | Flow (ml/min) BT | AT | AE |
|---|---|---|---|---|---|---|---|---|---|---|---|---|
| 8 | 122 | 122 | 120 | 3,05 | 3,23 | 3,00 | 3,39 | 3,56 | 3,39 | 88.75 | 1.30 | 91.50 |
| 9 | 118 | 123 | 124 | 3,13 | 3,12 | 3,18 | 3,40 | 3,43 | 3,45 | 86.75 | 87.50 | 81.00 |
| 10 | 121 | 121 | 123 | 3,05 | 3,05 | 3,12 | 3,42 | 3,44 | 3,42 | 87.25 | 91.25 | 87.50 |
| 11 | 120 | 124 | 127 | 3,16 | 3,08 | 3,28 | 3,49 | 3,43 | 3,52 | 83.50 | 29.00 | 83.50 |
| 12 | 123 | 121 | 124 | 3,04 | 3,18 | 3,18 | 4,43 | 3,44 | 3,42 | 84.25 | 70.00 | 85.00 |
| 13 | 121 | 123 | 124 | 3,05 | 3,10 | 3,22 | 3,42 | 3,43 | 3,45 | 85.00 | 45.00 | 89.50 |
| 14 | 122 | 124 | 127 | 3,08 | 3,20 | 3,07 | 3,46 | 3,47 | 3,38 | 85.00 | 73.50 | 92.00 |
| 15 | 122 | 120 | 125 | 3,09 | 3,11 | 3,21 | 3,37 | 3,42 | 3,45 | 86.25 | 85.50 | 84.00 |

BT—Before Treatment  AE—After Extraction
AT—After Treatment

TABLE XII (4 of 4)
Summary of test results

| Sample | Dye adsorption time to B.T. (min) BT | AT | AE | ΔP Characteristic* BT | AT | AE | Extraction (Mg/293 mm disc) |
|---|---|---|---|---|---|---|---|
| 8 | 6.30 | — | 32.88 | 0,138 | — | 0,145 | 76.8 |
| | | | | 0,138 | — | 2,1 | |
| 9 | 9.78 | 196.9 | 198.8 | 0,145 | 0,145 | 0,166 | |
| | | | | 2,1 | 0,72 | 1,69 | 85.2 |
| 10 | 7.86 | 79.60 | 73.39 | 0,145 | 0,138 | 0,152 | |
| | | | | 2,2 | 0,145 | 0,185 | 3.9 |
| 11 | 7.57 | 78.26 | 81.84 | 0,138 | 6,5 | 0,166 | |
| | | | | 2,0 | 0,185 | 0,394 | 68.4 |
| 12 | 7.24 | 28.60 | 11.99 | 0,138 | 0,173 | 0,145 | 109.6 |
| | | | | 2,0 | 0,152 | 0,166 | |
| 13 | 6.86 | 79.35 | 54.53 | 0,138 | 0,20 | 0,145 | |
| | | | | 2,0 | 0,152 | 0,185 | 54.0 |
| 14 | 7.34 | 22.03 | 14.95 | 0,138 | 0,138 | 0,152 | 110.0 |
| | | | | 2,0 | 0,152 | 0,166 | |
| 15 | 6.93 | 6.63 | 6.61 | 0,138 | 0,138 | 0,152 | 9.0 |
| | | | | 2,0 | 2,0 | 0,166 | |

BT—Before Treatment  AE—After Extraction
AT—After Treatment  B.T.—Breakthrough
*Upper Numbers are start of test and lower numbers are end of test (bar).

Summary

Samples 2, 4, 6, 8, 11, 12, 13 and 14, after treatment by the indicated mode exhibited a decrease in flow, i.e. the membranes exhibited clogged pores. This suggests that treatment with the polyamido-polyamine epichlorohydrin resins with a pH as diluted, i.e. less than 7, independent of pretreatment, exhibits pore clogging. After extraction (which tended to be high—less than 5 mg for 293 mm disc is acceptable for pharmaceutical uses) the flow rates increased indicating a reopening of the pores. Dye adsorption tests indicated the retention of charge after extraction.

Samples 1 and 3, which were treated with trimethylol melamine formaldehyde cationic resin (see aforementioned U.S. Serial No. 358,822 to *Ostreicher*) and cationic colloidal silica respectively, showed (a) marginal improvement in dye adsorption tests over untreated membrane, (b) intermediate levels of extraction and (c) even a decrease in dye adsorption after extraction (Sample 3). These tests indicate that the slight charge modification achieved was eliminated after extraction, i.e. the charge modifying agent was not bonded to the surface. Samples 5 and 7 indicate that charge modification, i.e. dye adsorption, after extraction is improved slightly after pretreatment with NaOH solution at pH 10.5, however, the charge modification is vastly inferior to the polyamido-polyamine epichlorohydrin charge modifying agents.

Samples 9 and 10 were both treated with polyamido-polyamine epichlorohydrin resin, adjusted to a pH of 10.5. Sample 10 was post-treated with tetraethylene pentamine. Both exhibited no clogging of pores after treatment. Sample 9 exhibited an unexpected enhancement of charge modification, which existed even after extraction over Sample 4 (or even 8) wherein the charge modifying agent was not pH adjusted. Sample 9, does however show an intermediate level of extraction. Sample 10 indicates the same unexpected enhancement of charge modification over Sample 4 (or even 8) but not as high. However, the extraction level is unexpectedly decreased to even below the untreated membrane (Sample 5) due to the post-treatment with the polyamine. Thus, preferred treatment mode for minimizing extraction levels is that of Sample 10. A preferred treatment mode for maximizing charge modification is that of Sample 9.

Example IX

Best mode

Two layers of wet microporous membrane, made as in Example IA, were laminated together and dried to 20—25% moisture. It has been found that membrane in such a wet, swollen condition absorbs charge modifying agents more efficiently than bone dry membrane.

The double-layer of membrane was then introduced into a 1.25% by weight solution of the polyamido-polyamine epichlorohydrin resin mentioned above. The pH of the bath was 10.5.

This bath was produced by diluting 17.17 Kg. of the polyamido-polyamine epichlorohydrin resin mentioned above from its initial 20% by weight concentration to 5% Five normal (5N) sodium hydroxide solution was then added to raise the pH to 10.5. The solution was then diluted with D.I. water having greater than 150,000 ohm-cm resistivity in a ratio (volume) 2.5:1. The total volume of bath solution was 60 gallons.

The membrane entered the bath of the polyamido-polyamine epichlorohydrin resin mentioned above at an angle of 30° from the horizontal to prevent bubble entrapment in the membrane which can prevent the charge modifying agent from diffusing into the membrane. The membrane was treated in this bath at a speed of 76.2 cm/min for a length of 121.9 cm.

Upon exiting this bath, the membrane was wiped on the bottom surface to remove excess water. A 3 minute air soak with cool air movement was used before the membrane entered the secondary charge modifying agent bath.

This bath was produced by adding 0.023% tetraethylene pentamine by weight .0513 kg 227. liters of D.I. water (at least 150,000 ohm-cm resistivity). The pH was about 9. The immersion conditions are identical to the first bath of primary charge modifying agent. The membrane was then wrapped around a take up roll.

The take up roll of wet membrane was stored for at least 3 hours. The roll was then dried at 121°C for 3 minutes to complete the reaction of the charge modifying agents.

The membrane was then washed in a subsequent operation and checked for extraction levels.

Example X

Membranes treated:

1) Millipore Durapore GVHP (0.2 um, hydrophobic)

2) Millipore Durapore GVWP (0.2 um, hydrophilic)

These membranes are microporous polyvinylidene difluoride, see *Grandine II* (U.S. Patent Nos. 4,203,847 and 4,203,848).

Treatment mode:

Contact with solutions:

Step 1: The polyamido-polyamine epichlorohydrin resin mentioned above, 2.0 wt.% solids in 20% (by volume) isopropyl/$H_2O$

Step 2: Tetraethylene pentamine, 0.23 wt.% solids in 20% (by volume) isopropyl/$H_2O$

Stretch dry at 75°C for 20 minutes.

| Sample | IBP (bar) | FAOP (bar) | Q (ml/min) | Dye adsorption—time to breakthru (min) | Extraction (mg per 47 mm disc) |
|---|---|---|---|---|---|
| GVHP untreated | 0,207 | 0,346 | 0 (at 15 PSID) | Not Run | 0.01 |
| GVHP treated | 0,376 | 1,035 | 0 (at 15 PSID) | Not Run | 0.14 |
| GVWP untreated | 3,52 | 3,69 | 72.0 | 1 | 0.47 |
| GVWP treated | 3,46 | 3,73 | 75.0 | 56.0 | 0.18 |

Q—flow of water

Conclusion: Hydrophobic polymer membranes are not amenable to charge modification by the methods of this invention whereas hydrophilic versions of the same polymer are amenable to charge modification.

Example XI

Cellulose ester based microporous membranes:

(1) Millipore Type MF—HA— mixed cellulose acetate and nitrate, .45 micron rated;

(2) Sartorius Type SM—11306 -cellulose nitrate, .45 micron rated; and

(3) Millipore Celotate—EH -cellulose acetate, .5 micron, were all treated with an aqueous solution containing the polyamido-polyamine epichlorohydrin resin mentioned above, .1 to .25% (by weight) tetraethylene pentamine and water (qs). The membranes were then dried at 80—90°C for 20—30 minutes. Tests (IBP, FAOP, flow tests and dye adsorption) indicate that significant charge modification had been achieved without deterioration in flow or bubble point characteristics. Although in the following claims the use of the membranes is claimed, it is not intended to claim the use of such membranes in medical treatment.

## Claims

1. A hydrophilic cationic charge modified microporous membrane comprising a hydrophilic organic polymeric microporous membrane having a micro-structure throughout said membrane and a primary charge modifying agent bonded to substantially all of the membrane micro-structure, without substantial pore size reduction or pore blockage, characterised in the primary charge modifying agent being a water soluble organic polymer having a molecular weight greater than about 1000, wherein each monomer thereof has at least one epoxide group capable of bonding to the surface of the membrane and at least one tertiary amine or quaternary ammonium group.

2. The microporous membrane of claim 1, characterised in a portion of the epoxy groups on the primary charge modifying agent are bonded to a secondary charge modifying agent selected from the group consisting of:

i) aliphatic amines which are polyamines having at least one primary amine or at least two secondary amines; and

ii) aliphatic amines having at least one secondary amine and a carboxyl or hydroxyl substituent.

3. The microporous membrane of claim 1 or 2, characterised in the hydrophilic organic polymeric microporous membrane is polyvinylidene fluoride.

4. The microporous membrane of claim 1 or 2, characterised in the hydrophilic organic polymeric microporous membrane is esters of cellulose.

5. The microporous membrane of claim 1 or 2, characterised in the hydrophilic organic polymeric microporous membrane is nylon.

6. The microporous membrane of claim 1 or 2, characterised in the hydrophilic organic polymeric microporous membrane is polyhexamethylene adipamide.

7. The microporous membrane of any of claims 1—6, characterised in the charged modified membrane is sanitized or sterilized.

8. The microporous membrane of any of claims 1—7, characterised in the primary charge modifying agent is a polyamido-polyamine epichlorohydrin resin.

9. The microporous membrane of any of claims 2—8, characterised in the secondary charge modifying agent is an amine of the formula:

$$H_2N-(R_1-\overset{\overset{H}{|}}{N}-)_x-R_2-NH_2$$

wherein $R_1$ and $R_2$ are alkyl of 1 to 4 carbon atoms and x is an integer from 0 to 4.

10. The microporous membrane of claim 9 characterised in the amine is tetraethylene pentamine of the formula:

$$H_2N-(C_2H_4-\overset{\overset{H}{|}}{N}-)_3-C_2H_4-NH_2$$

11. A process for producing a hydrophilic cationic modified microporous membrane comprising applying to a hydrophilic organic polymeric microporous membrane having a micro-structure throughout said membrane, a charge modifying amount of a primary cationic charge modifying agent bonded to substantially all of the membrane micro-structure, without substantial pore size reduction of pore blockage the primary charge modifying agent being a water soluble organic polymer having a molecular weight greater than about 1000, wherein each monomer thereof has at least one epoxide capable of bonding to the surface of the membrane and at least one tertiary amine or quaternary ammonium group.

12. The process of claim 11, characterised in a portion of the epoxy groups on the organic polymer are bonded to a secondary charge modifying agent selected from the group consisting of:

i) aliphatic amines which are polyamines having at least one primary amine or at least two secondary amines; and
ii) aliphatic amines having at least one secondary amine and a carboxyl or hydroxyl substituent.

13. The process of claims 11 and 12, characterised in applying the charge modifying agents by contacting the membranes with aqueous solutions of the charge modifying agents.

14. The process of claim 13, characterised in contacting the membrane first with the aqueous solution of the primary charge modifying agent and then with the aqueous solution of the secondary charge modifying agent.

15. The process of any of claims 11—14, characterised in subsequently sanitizing or sterilizing the microporous membrane.

16. The process of any of claims 11—14, characterised in the membrane is prepared by a process comprising:

a) preparing a dope solution of a nylon polymer in a solvent system comprising a mixture of at least one solvent and one non-solvent for the polymer, the amount of non-solvent being no greater than an amount required in induce nucleation of the solution to obtain a visible precipitate, said solvent being formic acid and said non-solvent being selected from the group consisting of methanol, methyl formate, water and glycerol;

b) directly casting said solution under the surface of a quenching bath comprising a non-solvent system for said polymer for a time sufficient to form micropores in said film, said non-solvent system being a mixture of methanol and water or formic acid and water.

17. The process of any of claims 13—16, characterised in the aqueous solution of the primary charge modifying agent contains at least about 1.0% agent by weight of the solution.

18. The process of claim 17, characterised in the aqueous solution of secondary charge modifying agent contains at least about .01% agent by weight of the solution.

19. The process of claim 17, characterised in the aqueous solution of the primary charge modifying agent contains less than about 5% agent by weight of the solution.

20. The process of claim 18, characterised in the aqueous solution of the secondary charge modifying agent contains less than about .5% agent by weight of the solution.

21. The process of any of claims 13—20, characterised in the weight ratio of the primary agent to the secondary agent in the aqueous solutions is from 2:1 to 500:1.

22. The process of claim 21, characterised in the weight ratio is from 25:1 to 75:1.

23. The process of any of claims 11—22, characterised in further chemically treating the nylon membrane to provide enhanced ninhydrin response evidencing free amino functionality, whereby the responsiveness to charge modification is improved.

24. The process of claim 23 characterised in the chemical treatment is performed prior to applying the primary charge modifying agent to the membrane or prior to contacting the membrane with the aqueous solution of primary charge modifying agent.

25. The process of claim 23, characterised in the chemical treatment is performed simultaneously

with applying the primary charge modifying agent to the membrane or simultaneously with contacting the membrane with the aqueous solution of primary charge modifying agent.

26. The process of claim 24, characterised in the membrane is chemically treated with an aqueous solution having a pH greater than about 7.

27. The process of claim 26, characterised in the pH is from 9 to 11.

28. The process of claim 27, characterised in the aqueous solution is an aqueous solution comprising sodium hydroxide.

29. The process of any of claims 11—28, characterised in further drying and curing the membrane.

30. Use of the membrane according to any of claims 1—10, for the filtration of parenteral or body liquids.

**Patentansprüche**

1. Hydrophile, mikroporöse Membran mit kationischer, modifizierter Ladung, umfassend eine hydrophile, organische, polymere, mikroporöse Membran mit einer Mikrostruktur über die gesamte Membran hinweg und ein primäres ladungsmodifizierendes Mittel, das überwiegend ohne eine wesentliche Porengrößenverringerung oder Porenblockierung an die gesamte Mikrostruktur der Membran gebunden ist, dadurch gekennzeichnet, daß das primäre ladungsmodifizierende Mittel ein wasserlösliches, organisches Polymer mit einem Molekulargewicht von über etwa 1 000 ist, wobei jedes seiner Monomeren mindestens eine Epoxidgruppe, die mit der Oberfläche der Membran eine Bindung eingehen kann und mindestens eine tertiäre Amin- oder quarternäre Ammoniumgruppe besitzt.

2. Mikroporöse Membran nach Anspruch 1, dadurch gekennzeichnet, daß ein Teil der Epoxygruppen an dem primären ladungsmodifizierenden Mittel an ein zweites ladungsmodifizierendes Mittel, gewählt aus der folgenden Gruppe, gebunden ist:

(i) aliphatische Amine, bei denen es sich um Polyamine mit mindestens einem primären Amin oder mindestens zwei sekundären Aminen handelt; und
(ii) aliphatische Amine mit mindestens einem sekundären Amin und einem Carboxyl- oder Hydroxylsubstituenten.

3. Mikroporöse Membran nach Anspruch 1 oder 2, dadurch gekennzeichnet, daß die hydrophile, organische, polymere, mikroporöse Membran Polyvinylidenfluorid ist.

4. Mikroporöse Membran nach Anspruch 1 oder 2, dadurch gekennzeichnet, daß die hydrophile, organische, polymere, mikroporöse Membran ein Zelluloseester ist.

5. Mikroporöse Membran nach Anspruch 1 oder 2, dadurch gekennzeichnet, daß die hydrophile, organische, polymere, mikroporöse Membran Nylon ist.

6. Mikroporöse Membran nach Anspruch 1 oder 2, dadurch gekennzeichnet, daß die hydrophile, organische, polymere, mikroporöse Membran Polyhexamethylenadipamid ist.

7. Mikroporöse Membran nach mindestens einem der Ansprüche 1 bis 6, dadurch gekennzeichnet, daß die ladungsmodifizierte Membran desinfiziert oder sterilisiert ist.

8. Mikroporöse Membran nach mindestens einem der Ansprüche 1 bis 7, dadurch gekennzeichnet, daß das primäre ladungsmodifizierende Mittel ein Polyamid-Polyamin-Epichlorhydrinharz ist.

9. Mikroporöse Membran nach mindestens einem der Ansprüche 2 bis 8, dadurch gekennzeichnet, daß das zweite ladungsmodifizierende Mittel ein Amin der folgenden Formel ist:

$$H_2N—(R_1—\overset{\displaystyle H \atop |}{N}—)_x—R_2—NH_2$$

worin $R_1$ und $R_2$ Alkyl mit 1 bis 4 Kohlenstoffatomen und x eine ganze Zahl von 0 bis 4 bedeuten.

10. Mikroporöse Membran nach Anspruch 9, dadurch gekennzeichnet, daß das Amin Tetraethylenpentamin der folgenden Formel ist:

$$H_2N—(C_2H_4—\overset{\displaystyle H \atop |}{N}—)_3—C_2H_4—NH_2$$

11. Verfahren zum Herstellen einer hydrophilen, mikroporösen Membran mit kationischer, modifizierter Ladung, umfassend das Aufbringen auf eine hydrophile, organische, polymere, mikroporöse Membran mit einer Mikrostruktur über die gesamte Membran hinweg einer ladungsmodifizierenden Menge eines primären, kationischen, ladungsmodifizierenden Mittels, das überwiegend an die gesamte Mikrostruktur der Membran ohne wesentliche Porengrößenverringerung oder Porenblockierung gebunden ist, wobei das primäre, ladungsmodifizierende Mittel ein wasserlösliches, organisches Polymer mit einem Molekulargewicht über etwa 1 000 ist, wobei jedes seiner Monomeren mindestens

eine Epoxidgruppe, die mit der Oberfläche der Membran eine Bindung eingehen kann und wenigstens eine tertiäre Amin- oder quaternäre Ammoniumgruppe besitzt.

12. Verfahren nach Anspruch 11, dadurch gekennzeichnet, daß ein Teil der Epoxygruppen an dem organischen Polymer an ein zweites ladungsmodifizierendes Mittel, gewählt aus der folgenden Gruppe, gebunden ist:

(i) aliphatische Amine, bei denen es sich um Polyamine mit mindestens einem primären Amin oder mindestens zwei sekundären Aminen handelt; und
(ii) aliphatische Amine mit mindestens einem sekundären Amin und einem Carboxyl- oder Hydroxylsubstituenten.

13. Verfahren nach den Ansprüchen 11 und 12, dadurch gekennzeichnet, daß das Aufbringen der ladungsmodifizierenden Mittel dadurch erfolgt, daß man die Membranen mit wäßrigen Lösungen der ladungsmodifizierenden Mittel in Berührung bringt.

14. Verfahren nach Anspruch 13, dadurch gekennzeichnet, daß man die Membran zuerst mit der wässrigen Lösung des primären ladungsmodifizierenden Mittels und dann mit der wässrigen Lösung des zweiten ladungsmodifizierenden Mittels in Berührung bringt.

15. Verfahren nach mindestens einem der Ansprüche 11 bis 14, dadurch gekennzeichnet, daß man die mikroporöse Membran anschließend desinfiziert oder sterilisiert.

16. Verfahren nach mindestens einem der Ansprüche 11 bis 14, dadurch gekennzeichnet, daß die Membran durch ein Verfahren hergestellt wird, das die folgenden Schritte umfaßt:

(a) Herstellen einer Impf- bzw. Tränklösung eines Nylonpolymeren in einem Lösungsmittelsystem, umfassend ein Gemisch aus mindestens einem Lösungsmittel und einem Nicht-Lösungsmittel für das Polymer, wobei die Menge des Nicht-Lösungsmittels nicht größer ist als eine zum Induzieren der Keimbildung der Lösung zur Erzielung eines sichtbaren Niederschlags notwendigen Menge, wobei das Lösungsmittel Ameisensäure ist und das Nicht-Lösungsmittel aus der Gruppe Methanol, Methylformiat, Wasser und Glycerin, ausgewählt wird;

(b) direktes Gießen der Lösung unter die Oberfläche eines Abschreckbades, enthaltend ein Nicht-Lösungsmittelsystem für das Polymer, für eine ausreichende Zeit zur Bildung von Mikroporen in dem Film, wobei das Nicht-Lösungsmittelsystem eine Mischung aus Methanol und Wasser oder Ameinensäure und Wasser ist.

17. Verfahren nach mindestens einem der Ansprüche 13 bis 16, dadurch gekennzeichnet, daß die wäßrige Lösung des primären ladungsmodifizierenden Mittels mindestens etwa 1,0% des Mittels, bezogen auf das Gewicht der Lösung, enthält.

18. Verfahren nach Anspruch 17, dadurch gekennzeichnet, daß die wässrige Lösung des zweiten ladungsmodifizierenden Mittels mindestens etwa 0,01% des Mittels, bezogen auf das Gewicht der Lösung, enthält.

19. Verfahren nach Anspruch 17, dadurch gekennzeichnet, daß die wässrige Lösung des primären ladungsmodifizierenden Mittels weniger als etwa 5% des Mittels, bezogen auf das Gewicht der Lösung, enthält.

20. Verfahren nach Anspruch 18, dadurch gekennzeichnet, daß die wässrige Lösung des zweiten ladungsmodifizierenden Mittels weniger als etwa 0,5% des Mittels, bezogen auf das Gewicht der Lösung, enthält.

21. Verfahren nach mindestens einem der Ansprüche 13 bis 20, dadurch gekennzeichnet, daß das Gewichtsverhältnis des primären Mittels zu dem zweiten Mittel in den wässrigen Lösungen 2:1 bis 500:1 beträgt.

22. Verfahren nach Anspruch 21, dadurch gekennzeichnet, daß das Gewichtsverhältnis 25:1 bis 75:1 beträgt.

23. Verfahren nach mindestens einem der Ansprüche 11 bis 22, dadurch gekennzeichnet, daß man weiterhin die Nylonmembran chemisch behandelt, um ein verstärktes Ansprechen auf Ninhydrin, was freie Aminofunktionalität anzeigt, vorzusehen, wodurch das Ansprechvermögen auf Ladungsveränderung verbessert wird.

24. Verfahren nach Anspruch 23, dadurch gekennzeichnet, daß die chemische Behandlung vor dem Aufbringen des primären ladungsmodifizierenden Mittels auf die Membran oder vor dem Kontaktieren der Membran mit der wässrigen Lösung des primären ladungsmodifizierenden Mittels durchgeführt wird.

25. Verfahren nach Anspruch 23, dadurch gekennzeichnet, daß die chemische Behandlung gleichzeitig mit dem Aufbringen des primären ladungsmodifizierenden Mittels auf die Membran oder gleichzeitig mit dem Kontaktieren der Membran mit der wässrigen Lösung des primären ladungsmodifizierenden Mittels durchgeführt wird.

26. Verfahren nach Anspruch 24, dadurch gekennzeichnet, daß die Membran chemisch mit einer wässrigen Lösung mit einem pH über etwa 7 behandelt wird.

27. Verfahren nach Anspruch 26, dadurch gekennzeichnet, daß der pH 9 bis 11 beträgt.

28. Verfahren nach Anspruch 27, dadurch gekennzeichnet, daß die wässrige Lösung eine Natriumhydroxid enthaltende, wässrige Lösung ist.

29. Verfahren nach mindestens einem der Ansprüche 11 bis 28, dadurch gekennzeichnet, daß man die Membran weiterhin trocknet und härtet.

30. Verwendung der Membran nach einem der Ansprüche 1 bis 10 zum Filtrieren von parenteralen Flüssigkeiten oder Körperflüssigkeiten.

**Revendications**

1. Membrane microporeuse hydrophile modifiée par une charge cationique comprenant une membrane microporeuse polymère organique hydrophile ayant une micro-structure dans toute ladite membrane et un agent modificateur de charge primaire lié à la quasi-totalité de la microstructure de la membrane, sans diminution notable de la taille des pores ou obturation des pores, caractérisée en ce que l'agent modificateur de charge primaire est un polymère organique soluble dans l'eau ayant une masse moléculaire supérieure à environ 1000, chacun de ses monomères ayant au moins un groupe époxyde capable de se lier à la surface de la membrane et au moins un groupe amine tertiaire ou ammonium quaternaire.

2. Membrane microporeuse selon la revendication 1, caractérisée en ce qu'une partie des groupes époxydes sur l'agent modificateur de charge primaire est liée à un agent modificateur de charge secondaire choisi parmi:

i) des amines aliphatiques qui sont des polyamines ayant au moins une amine primaire ou au moins deux amines secondaires; et
ii) des amines aliphatiques ayant au moins une amine secondaire et un substituant carboxyle ou hydroxyle.

3. Membrane microporeuse selon la revendication 1 ou la revendication 2, caractérisée en ce que la membrane microporeuse polymère orgénique hydrophile est un fluorure de polyvinylidène.

4. Membrane microporeuse selon la revendication 1 ou 2, caractérisée en ce que la membrane microporeuse organique organique hydrophile est constituée par des esters de cellulose.

5. Membrane microporeuse selon la revendication 1 ou 2, caractérisée en ce que la membrane microporeuse polymère organique hydrophile est un nylon.

6. Membrane microporeuse selon la revendication 1 ou 2, caractérisée en ce que la membrane microporeuse polymère organique hydrophile est constituée par du polyhéxaméthylène adipamide.

7. Membrane microporeuse selon l'une quelconque des revendications 1 à 6, caractérisée en ce que la membrane modifiée par une charge est aseptisée ou stérilisée.

8. Membrane microporeuse selon l'une quelconque des revendications 1 à 7, caractérisée en ce que l'agent modificateur de charge primaire est une résine de polyamido-polyamine épichlorhydrine.

9. Membrane microporeuse selon l'une quelconque des revendications 2 à 8, caractérisée en ce que l'agent modificateur de charge secondaire est une amine répondant à la formule:

$$H_2N{-}(R_1{-}\overset{\displaystyle H}{\overset{|}{N}}{-})_x{-}R_2{-}NH_2$$

dans laquelle $R_1$ et $R_2$ sont des groupes alkyles de 1 à 4 atomes de carbone et x est un entier de 0 à 4.

10. Membrane microporeuse selon la revendication 9, caractérisée en ce que l'amine est la tétraéthylènepentamine répondant à la formule:

$$H_2N{-}(C_2H_4{-}\overset{\displaystyle H}{\overset{|}{N}}{-})_3{-}C_2H_4{-}NH_2$$

11. Procédé pour produire une membrane microporeuse modifiée par une charge cationique consistant à appliquer à une membrane microporeuse polymère organique hydrophile ayant une micro-structure dans toute ladite membrane, une quantité modificatrice de la charge d'un agent modificateur de charge cationique lui-même, lié à la quasi-totalité de la micro-structure de la membrane, sans diminution notable de la taille des pores ou obturation des pores, l'agent modificateur de charge polymère étant un polymère soluble dans l'eau ayant une masse moléculaire supérieure à environ 1000, chacun de ses monomères ayant au moins un groupe époxyde capable de se lier à la surface de la membrane et au moins un groupe amine tertiaire ou un groupe ammonium quaternaire.

12. Procédé selon la revendication 11, caractérisé en ce qu'une partie des groupes époxydes sur le polymère organique sont liés à un agent modificateur de charge secondaire choisi parmi:

i) des amines aliphatiques qui sont des polyamines ayant au moins une amine primaire ou au moins deux amines secondaires, et
ii) des amines aliphatiques ayant au moins une amine secondaire et un substituant carboxyle ou hydroxyle.

13. Procédé selon les revendications 11 et 12, caractérisé par l'application des agents modificateurs de charge par mise en contact des membranes avec des solutions aqueuses des agents modificateurs de charge.

14. Procédé selon la revendication 13, caractérisé par la mise en contact de la membrane en premier lieu avec la solution aqueuse de l'agent modificateur de charge primaire puis avec la solution aqueuse de l'agent modificateur de charge secondaire.

15. Procédé selon l'une quelconque des revendications 11 à 14, caractérisé en ce qu'on aseptise ou stérilise ensuite la membrane microporeuse.

16. Procédé selon l'une quelconque des revendications 11 à 14, caractérisé en ce que la membrane est préparée par un procédé comprenant:

a) la préparation d'une solution d'addition d'un polymère de nylon dans un système solvant comprenant un mélange d'au moins un solvant et d'un liquide non solvant vis-à-vis du polymère, la quantité du liquide non solvant n'étant pas supérieure à une quantité nécessaire pour induire une nucléation de la solution pour obtenir un précipité lisible, ledit solvant étant de l'acide formique et ledit liquide non solvant étant choisi parmi le méthanol, le formiate de méthyle, l'eau et le glycérol;

b) la coulée directe de ladite solution sous la surface d'un bain de trempage comprenant un système non solvant pour ledit polymère pendant un temps suffisant pour former des micropores dans ladite pellicule, ledit système non solvant étant un mélange de méthanol et d'eau ou d'acide formique et d'eau.

17. Procédé selon l'une quelconque des revendications 13 à 16, caractérisé en ce que la solution aqueuse de l'agent modificateur de charge primaire contient au moins environ 1,0% en poids d'agent par rapport à la solution.

18. Procédé selon la revendication 17, caractérisé en ce que la solution aqueuse de l'agent modificateur de charge secondaire contient au moins environ 0,01% d'agent en poids par rapport à la solution.

19. Procédé selon la revendication 17, caractérisé en ce que la solution aqueuse de l'agent modificateur de charge primaire contient moins d'environ 5% d'agent en poids par rapport à la solution.

20. Procédé selon la revendication 18, caractérisé en ce que la solution aqueuse de l'agent modificateur de charge secondaire contient moins d'environ 0,5% d'agent en poids par rapport à la solution.

21. Procédé selon l'une quelconque des revendications 13 à 20, caractérisé en ce que le rapport pondéral de l'agent primaire à l'agent secondaire dans les solutions aqueuses est de 2:1 à 500:1.

22. Procédé selon la revendication 21, caractérisé en ce que le rapport pondéral est de 25:1 à 75:1.

23. Procédé selon l'une quelconque des revendications 11 à 22, caractérisé en outre en ce qu'on traite chimiquement la membrane de nylon pour fournir une réaction renforcée à la ninhydrine mettant en évidence la fonctionalité amino libre, cette possibilité de réaction à la modification de charge étant améliorée.

24. Procédé selon la revendication 23, caractérisé en ce que le traitement chimique s'effectue avant application de l'agent modificateur de charge primaire sur la membrane ou avant mise en contact de la membrane avec la solution aqueuse d'agent modificateur de charge primaire.

25. Procédé selon la revendication 23, caractérisé en ce que la traitement chimique s'effectue en même temps que l'application de l'agent modificateur de charge primaire sur la membrane ou en même temps que la mise en contact de la membrane avec la solution aqueuse d'agent modificateur de charge primaire.

26. Procédé selon la revendication 24, caractérisé en ce que la membrane est chimiquement traité par une solution aqueuse ayant un pH supérieur à environ 7.

27. Procédé selon la revendication 26, caractérisé en ce que le pH est compris entre 9 et 11.

28. Procédé selon la revendication 27, caractérisé en ce que la solution aqueuse est une solution aqueuse comprenant de l'hydroxyde de sodium.

29. Procédé selon l'une quelconque des revendications 11 à 28, caractérisé en outre en ce qu'on sèche et durcit la membrane.

30. Utilisation de la membrane selon l'une quelconque des revendications 1 à 10 pour filtration de liquides parentéraux ou corporels.

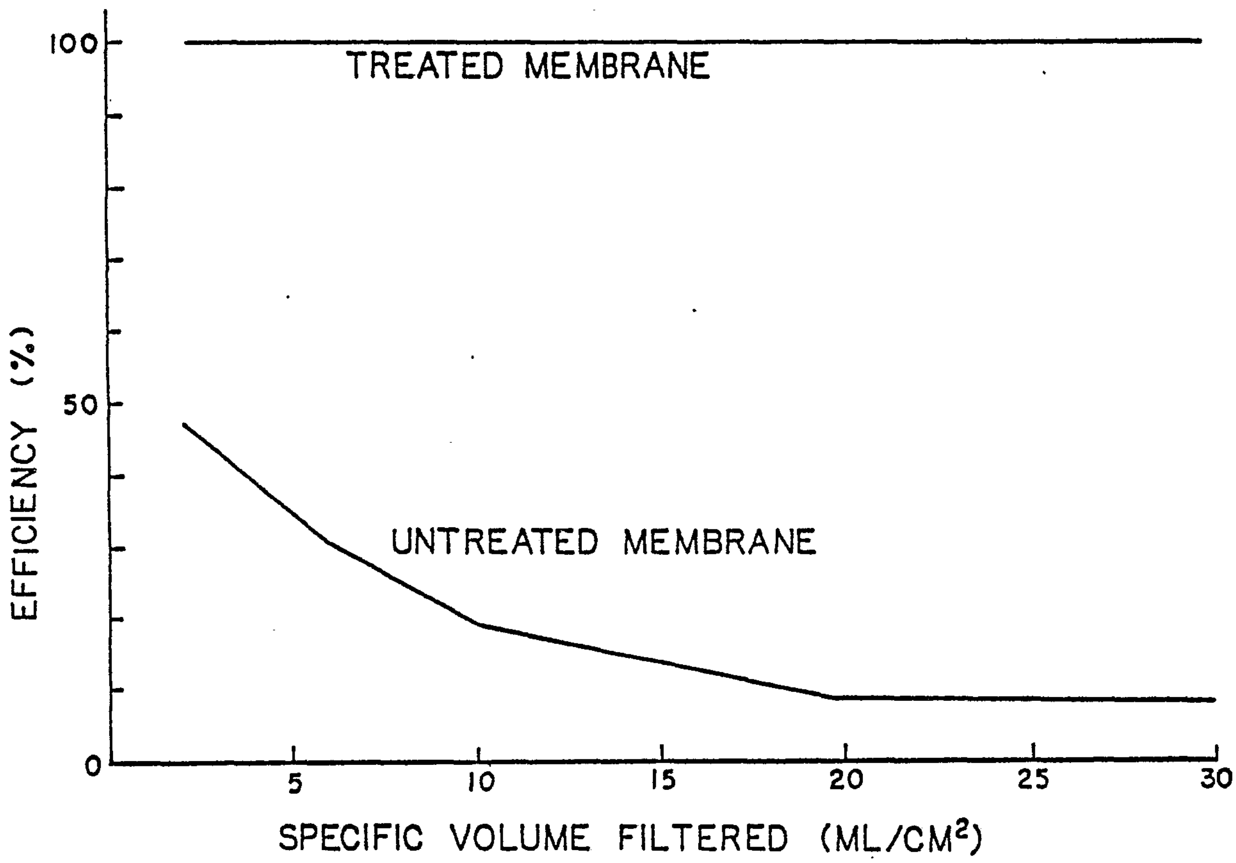
100
50
0
TREATED MEMBRANE
UNTREATED MEMBRANE
EFFICIENCY (%)
5
10
15
20
25
30
SPECIFIC VOLUME FILTERED (ML/CM2)

FIG. 1

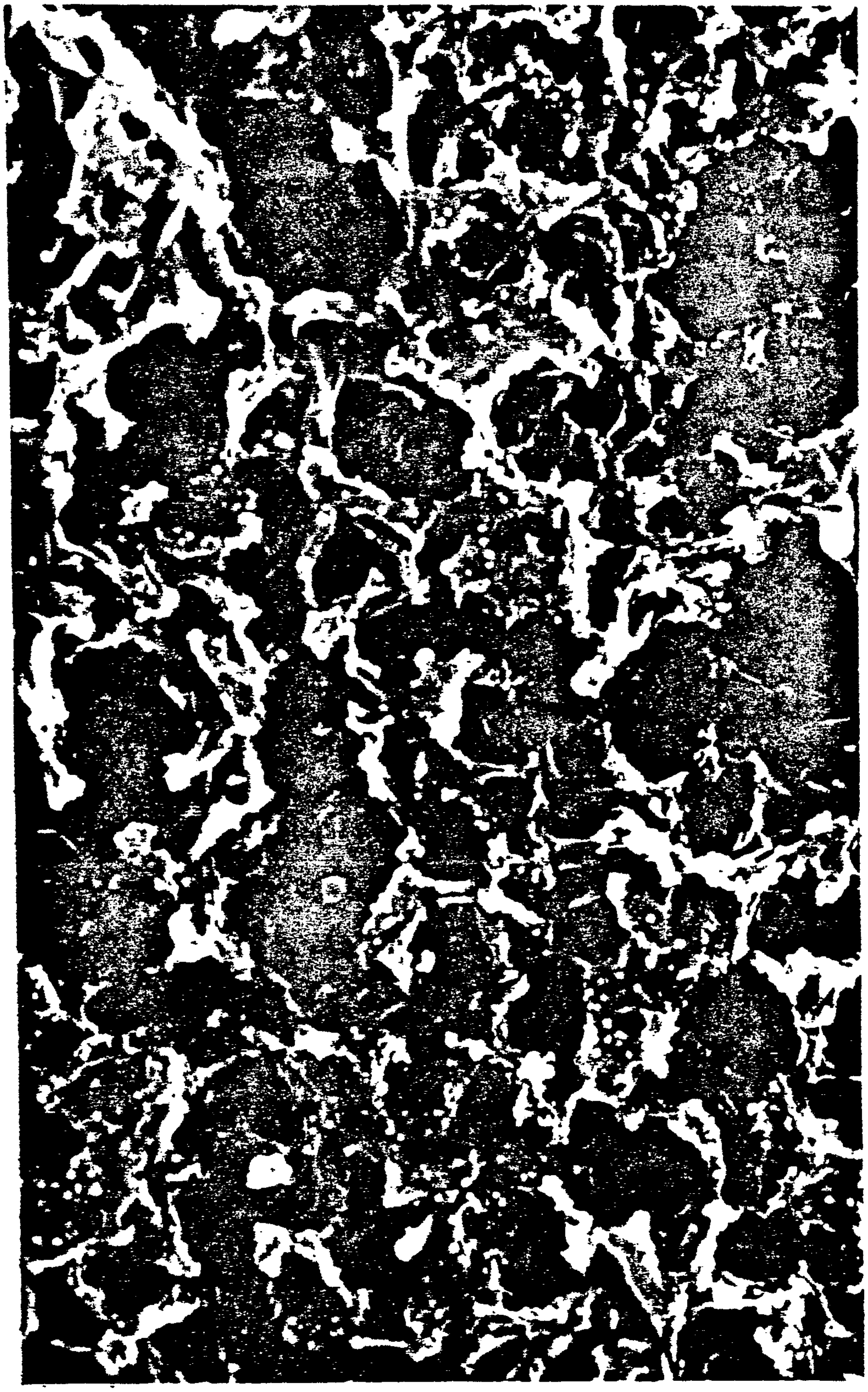

9000 MAGNIFICATION

FIG. 2

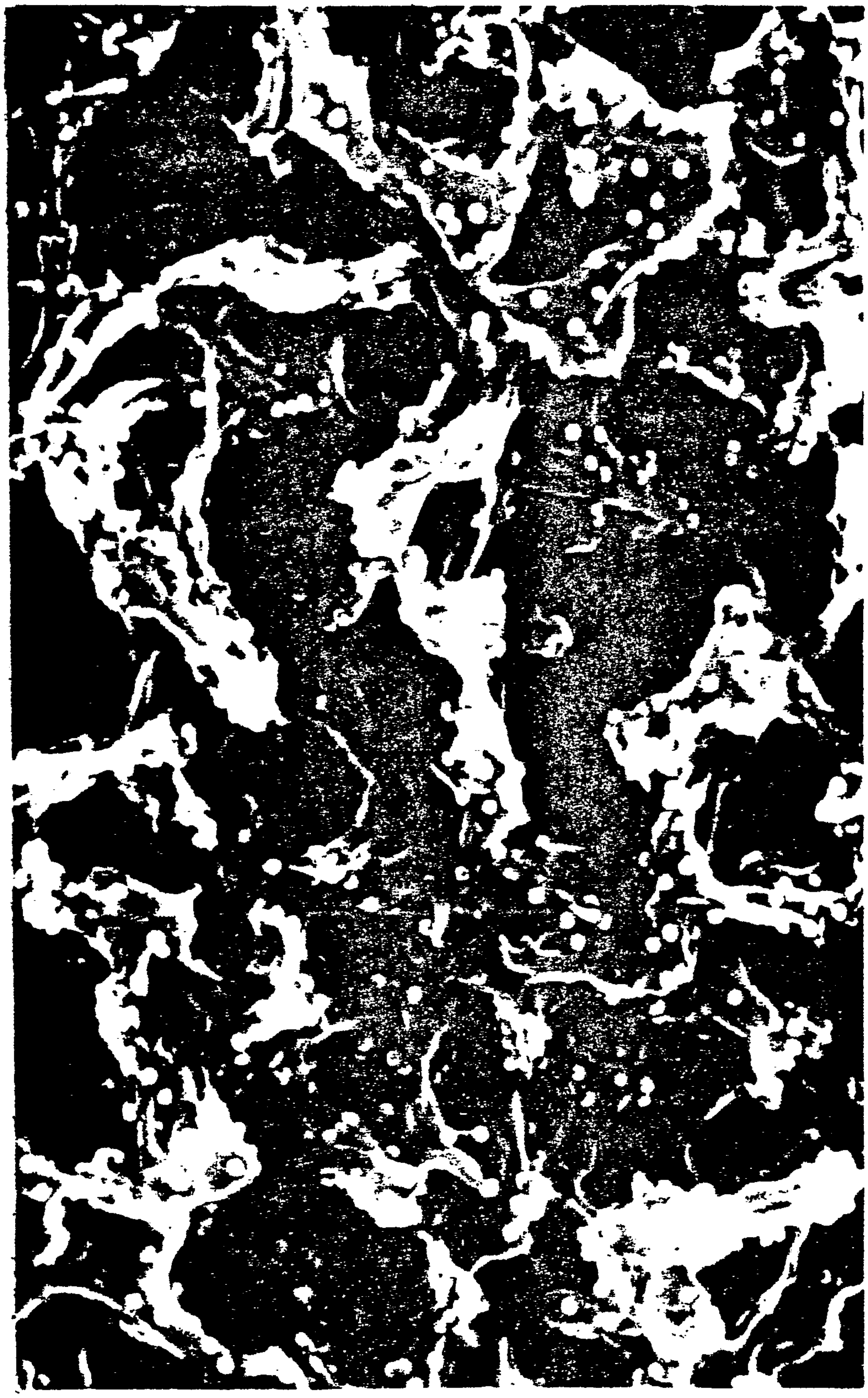

18000 MAGNIFICATION

FIG. 3